# EUROPEAN PATENT SPECIFICATION

(11) **EP 0 552 949 B1**
(45) Date of publication and mention of the grant of the patent: **25.03.1998**
(21) Application number: 93300413.7
(22) Date of filing: 21.01.1993
(51) Int. Cl.: A61F 2/34

(54) **Process for the manufacture of an acetabular cup for a total hip prosthesis**
Verfahren zur Herstellung einer Hüftgelenkpfanne für eine Hüfttotalprothese
Procédé pour la fabrication d'une cupule acétabulaire pour une prothèse totale de la hanche

(30) Priority: 23.01.1992 GB 9201477
(43) Date of publication of application: 28.07.1993
(73) Proprietor: HOWMEDICA INTERNATIONAL INC., Shannon Co. Clare (IE)
(72) Inventor: Rushton, Neil, Cambridge CB2 2AW (GB); Field, Richard Eddy, London N8 7HL (GB); Nuijten, Peter, Patrickswell, County Limerick (IE)
(74) Representative: Bridge-Butler, Alan James

(56) References cited:
- EP-A- 0 051 729
- EP-A- 0 187 881
- EP-A- 0 331 622
- EP-A- 0 388 745
- FR-A- 2 598 908
- FR-A- 2 651 675
- FR-A- 2 653 659
- US-A- 2 910 978

## Description

This invention relates to a process for the manufacture of an acetabular cup for a total hip prosthesis and total hip prostheses embodying such cups.

Consideration of the acetabulum in the human pelvis in the load bearing area shows that the load from the femur is transmitted and passes in a relatively direct line from that area up to the sacrum via a bar of trabeculae or a column of trabecular bone which is substantially straight so that in an X-ray of a human being standing it can be seen that there is a direct continuation of the medial compressive system of the proximal femur. it is therefore desirable that this area of the acetabulum is loaded and the remainder of the acetabulum should not have any load transmitted to the underlying bowl and that any further articular surface beyond the load bearing area, which is substantially horse-shoe shaped can only be justified if for some reason the articulation should be greater for the stability of the femoral head. This means that with a large headed prosthesis the surface area of contact can be used down to levels closer to that of a conventional stem head cup arthroplasty.

In the mid 1970's total hip replacement designs were made which were classified as double cup arthroplasties. In these the femoral component had no intramedullary stem but used a relatively thin part-spherical shell which was placed over the top of the femur from which a minimum of bone was removed. Inevitably, the bearing surface for such a design was large, approximating to the normal anatomy. The acetabular component bearing surface also had to be large and there was little opportunity to remove a significant quantity of bone from the acetabulum. The acetabular cups for such designs therefore have thin wall thicknesses and are inherently flexible. At the period of development of these designs almost all acetabular cups where cemented into position and these thin walled flexible acetabular cups flexed too much, thereby causing cracks which progressed around the bones cement mantle or through it, leading ultimately to a loose implant.

There tends to be elastic movement in the acetabulum which causes distortion or deformation under the load.

Typical examples of such total hips are shown in US Patent Specification No. 4 123 806, and French Patent Application No. 2 361 861 (76 25215).

FR-A-2 598 908 shows a construction comprising a backing and an inner bearing component which are separate members. The backing comprises a main part-spherical portion and anterior and posterior horns extending therefrom. The backing cup is intended to be fixed by impact and by screws and in the construction described in the specification is said to be made from a metal, titanium, although other materials could be used. The specification instructs the reader to first position the cup and locate it in place and then insert the liner which can be made from polyethylene. Thus, the liner and cup are separate parts and this can lead to wear and loosening in use.

The present invention therefore is intended to provide a process for the manufacture of an acetabular cup for surface replacement and which can be thin walled, at the same time having the ability to flex in harmony with bone movements without this leading to loosening of the implant. Bone cements at present in use are not well suited to accommodate these movements, but it is possible that more flexible materials may be found in the future.

The present process is intended to provide an acetabular cup which can not only be used for large bearing surface diameters but can also be used with smaller bearing surfaces of more conventional diameters, for example 28 mm and 32 mm.

According to the present invention a process for the manufacture of an acetabular cup for a total hip prosthesis which includes an outer backing and an inner bearing component, said backing comprising a substantially part spherical main portion and two independent arms projecting therefrom and formed by a separation or opening in the rim of said backing is characterised in that said backing and said inner bearing component are made from synthetic plastics material and are moulded together to form a single component.

As mentioned above, the backing is preferably sufficiently flexible to absorb acetabular deformation of the pelvis of the user.

The backing can be stiffer than the inner bearing component and in a preferred construction the inner bearing surface of the bearing component is substantially part-spherical over a portion thereof spaced substantially opposite the separation or opening between the arms of the backing and is relieved over its remaining inner bearing surface, said relief being tangential at the junction with the said part spherical portion.

The bearing component can take various forms, for example, it can have independent arms similar to the backing with a separation or opening between them and can be of substantially the same configuration as the backing or it can be substantially hemispherical and extend across the separation or opening between the arms of the backing.

The invention can be performed in various ways and some embodiments will now be described by way of example and with reference to the accompanying drawings in which :
Figure 1 is a diagrammatic view of part of a human pelvis;
Figure 2 is a plan view from below of an acetabular cup according to the invention;
Figure 3 is an isometric view of an alternative cup construction;
Figure 4 is a side elevation of the acetabular cup shown in Figure 3;
Figure 5 is an end elevation of the cup shown in Figure 3;
Figure 6 is a plan view from below of the cup shown in Figures 3, 4 and 5;
Figure 7 - is a cross sectional view on the line VII-VII of Figure 4;
Figure 8 is an isometric view of a modification of the construction shown in Figures 3 to 7;
Figure 9 is a side elevation of an alternative construction of cup;
Figure 10 is an end elevation of the cup shown in Figure 8;
Figure 11 is a plan view from below of the cup shown in Figures 9 and 10; and,
Figure 12 is a cross sectional end elevation taken on the line XII-XII of Figure 9.

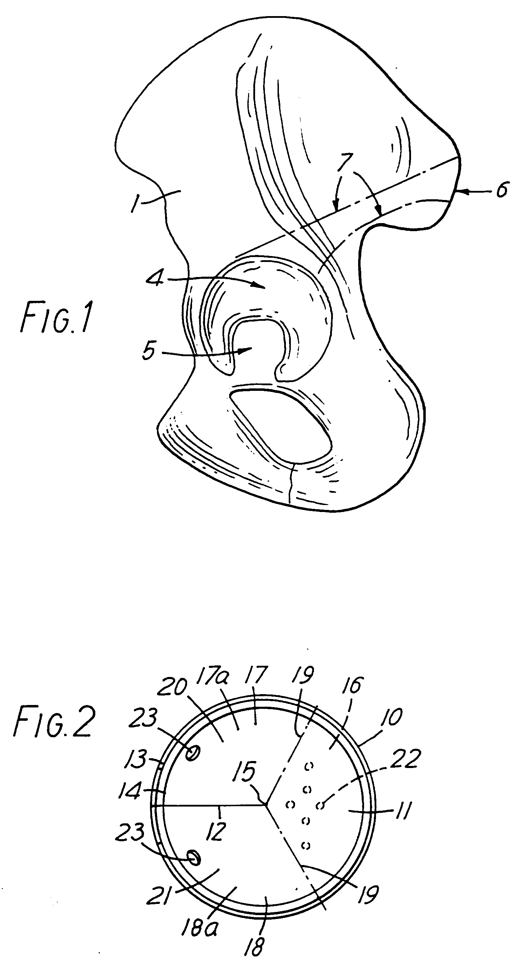

Figure 1 is a diagrammatic view of half of a human pelvis 1 showing the acetabulum 4. Deformation of the acetabulum 4 occurs during walking and hip joint loading. The acetabulum 4 is roughly a horse-shoe shaped cup with a depressed portion which constitutes the foveal recess 5. The articulation with the spine (the sacroiliac joint) is indicated at 6.

The main column of support which transfers loads from the acetabulum 4 through the ilium 1 up to the sacroiliac (spinal) joint 6 is indicated by chain lines 7.

Figure 2 is a plan view of an acetabular cup according to the present invention, which comprises an outer backing 10 which is substantially hemispherical and an inner bearing component 11 of substantially the same shape and which fits within the backing 10. The backing can be made of any suitable material, for example, a carbon fibre reinforced plastics material and the inner bearing component from a suitable plastics bearing material. Both the backing and bearing components are separated along a line 12 which extends from their outer rims, respectively 13 and 14 to approximately the centre 15 of the cup. This produces a main portion 16 of the backing 10 which is substantially part-spherical and two independent arms 17 and 18 which extend from the main part 16. The external shape of the inner bearing component 11 is also hemispherical and fits within the backing 10 but the inner surface of the bearing component is only hemispherical over its main portion up to the chain lines 19, from there it is relieved over the inner surfaces 17a and 18a of its arms 20 and 21.

In order to locate this cup within the acetabular socket of the patient projections in the form of spikes 22 are provided on the outer surface of the backing and holes 23 are formed at the end of each arm 17 and 18 through which pins or screws can be placed to hold the arms in position, The holes 23 and 24 extend through both the backing and of the bearing component.

The cup is placed in a prepared acetabular socket with the separation line 12 extending downwardly substantially in line with the foveal recess 5 so that the main loads are carried upwardly through the main portion 16 of the backing and any deformation of the acetabulum can be accommodated by separation line 12 between the arms 17 and 18.

In the arrangement shown the cup is applied for use with a physiological bearing surface typically 40 mm to 60 mm but the cup could be made for more normal total hip bearing diameters down to 22 mm.

The relieved surfaces on the inner bearing component allow for flexibility when co-operating with the femoral component (not shown). The main portion 16 of the inner bearing component is spherically machined to be a very close fit with the diameter of the co-operating metal femoral component, but to avoid creating a binding fit at the bearing surface the two arms are relieved as described above.

The relieving can be done by a number of alternative geometrical configurations, for example, the same spherical radius could be chosen for the three compartments but different centres of rotation can be chosen for each or alternatively a larger radius could be chosen on the same centre for the two arms, Again a slightly larger radius can be used with an orbiting centre of rotation to provide a relief which is tangential at the junction with the curvature of the main portion 16. It is important that there is a clearance area round the equator of the cup the femoral component is bearing on the main portion of the cup, for example when walking, but when unusual forces come into play, for example when rising from a seat, then bearing can occur in the clearance area as either of the two arm portions have adequate contact area.

The moulded backing is moulded to the bearing portion so that the baring portion extends somewhat to provide an under cut ridge around the rim.

The spikes at 22 are intended to resist excessive slide movement between the implant and the bone.

The intention of the designs is to transfer load into the pelvis in as physiological a way as possible and so that load is not transferred to the lower parts of the acetabulum but is pointed directly along the lines 7 as shown in Figure 1.

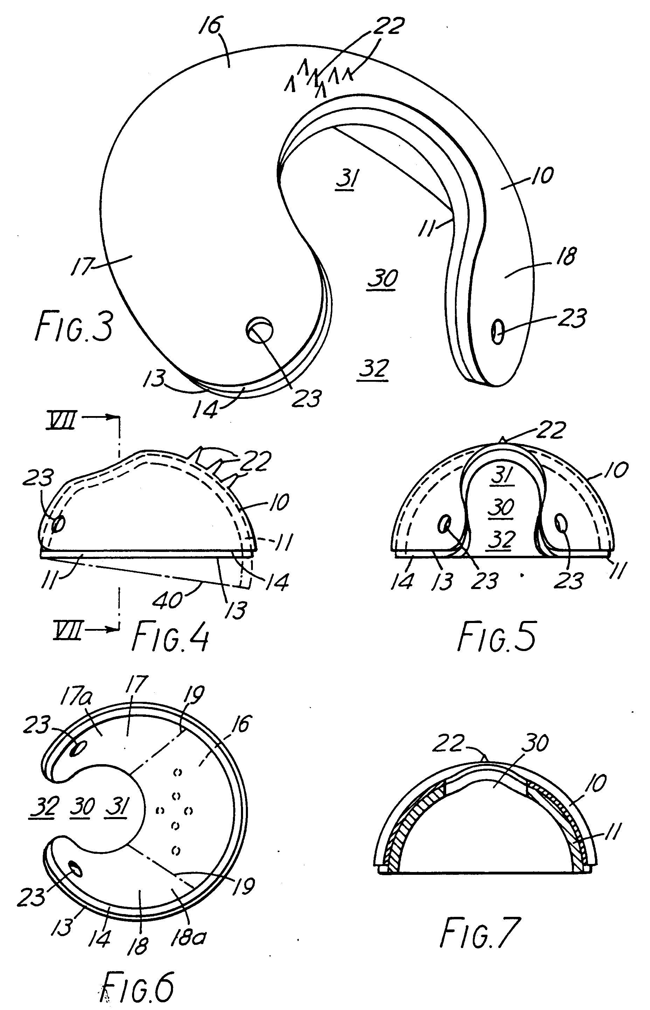

In the construction shown in Figures 3, 4, 5, 6 and 7 similar reference numerals are used to indicate similar parts. In this arrangement however the arms 17 and 18 are spaced apart to provide a gap or opening 30 between them. As will be seen the arms are spaced apart about an arc on the part-spherical main portion 16 breaking out on the rim 14 and the arms themselves and the main portion are together substantially part-spherical.

The backing thus comprises a substantially part-spherical wall having a rim which is interrupted by a shaped opening to provide the two spaced apart arms 17 and 18. In fact the rim is extended inwardly around the opening.

The main part of the opening 31 is substantially semicircular and has a mouth 32 which provides the interruption in the rim and which is of smaller width than the remainder 31 of the opening. The backing is therefore substantially horse-shoe shaped.

The spikes 22 are provided on the backing itself but in an alternative construction they could be provided on the inner bearing component and extend through apertures in the backing.

The backing is sufficiently flexible to accept deformation of the acetabulum of the patient, but it is usually stiffer than the inner bearing component.

As with the arrangement shown in Figure 2 the backing and the bearing component are made from synthetic plastics material and are moulded together.

The bearing surface of the bearing component is again relieved as described with regard to Figure 2.

With the arrangement described above the outside diameter on the fixation surface is 59 mm and the inside bearing surface is 50 mm.

It has been found that this particular shape of opening is convenient and successful and the load is transferred in to the pelvis as required, in particular, this shape of opening ensures efficiently that no load is transferred in to the bone at undesired locations.

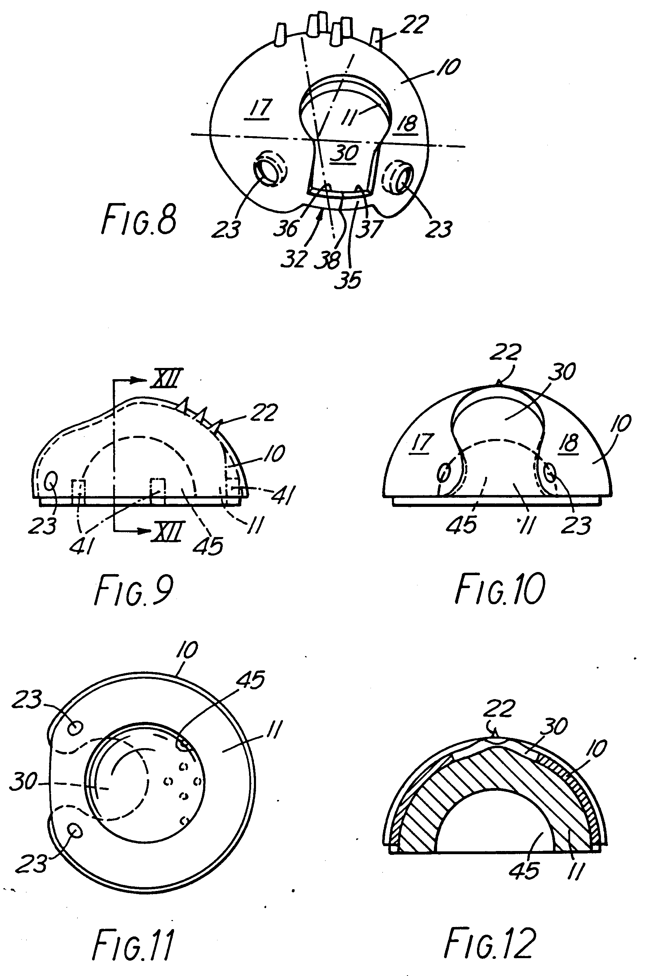

Figure 8 shows a modified form of the construction shown in Figures 3, 4, 5, 6 and 7 and the same reference numerals are used to indicate similar parts. In this construction, to prevent collapse of the horse-shoe shaped components as it is pressed into the acetabulum, a divided spacer bar 35 is provided which bridges the mouth 32 of the opening 30 where the opening meets the equatorial rim of the backing. It will be seen that the bar 35 comprises two extensions 36 and 27 respectively on the arms 17 and 18 and the abutting ends contact each other at the line 38. By providing the bar in two parts the arms of the horse-shoe shaped backing can deflect open but collapse is resisted as the ends of the extensions 36 and 37 come into direct abutment on the line 38.

Figures 9, 10, 11 and 12 show another choice of construction which is intended for use for a more normal total hip bearing diameter down to 22 mm. One again in these Figures the same reference numerals are used to indicate similar parts to those shown in Figures 4, 5, 6, 7 and 8. In this construction the inner bearing component 11 is not provided with an opening or a slot and it is substantially hemispherical, thus, the inner bearing surface 45 is unbroken. When making this type of device and moulding the parts together it is possible for the opening 30 to be filled by the material from the bearing component 11. Load transfer is minimised or eliminated however from the boss which forms within the opening by recessing the boss so it is not flush with the outer surface of the backing, moreover, the modulus of elasticity of the bearing surface is arranged to be lower than that of the surrounding backing and therefore the increased rigidity of the backing will cause the load to be preferentially transferred to the bone through this more rigid portion.

A hood or skirt could be provided on the inner bearing component and such a feature is indicated by broken lines 40 in Figure 4. Bearing inserts carrying such feature are known in themselves and are shown, for example, in European Patent Application No. 90313400.5 (publication number 0 436 317). As such constructions are known they will not be described herein in further detail.

## Claims

1. A process for the manufacture of an acetabular cup for a total hip prosthesis including an outer backing (10) and an inner bearing component (11), said backing (10) comprising a substantially part-spherical main portion and two independent arms (17,18) projecting therefrom and formed by a separation (12) or opening (30) in the rim (13) of said backing (10) characterised in that said backing (10) and said inner bearing component (11) are made from synthetic plastics material and are moulded together to form a single component.

2. A process as claimed in claim 1 characterised in that said backing (10) is stiffer than the inner bearing component (11).

3. A process as claimed in claim 1 and claim 2 characterised in that the inner bearing surface of the bearing component is substantially part-spherical over a portion (16) thereof spaced substantially opposite the separation (12) or opening (30) between the arms of the backing (17,18) and is relieved over its remaining inner bearing surface (17a,18a), said relief being tangential at the junction with the said part spherical portion.

4. A process as claimed in claim 3 characterised in that said relieving is formed as a larger radius of cut than said spherical portion (16) and which has been orbited around a centre of rotation.

5. A process as claimed in claims 1 to 4 characterised in that the bearing component (11) has independent arms (20,21) similar to the backing (10) with a separation (12) or opening (30) between them.

6. A process as claimed in claim 5 characterised in that said bearing component (11) is of substantially the same configuration as the backing (10).

7. A process as claimed in claims 1 to 4 characterised in that the bearing component (11) is substantially hemispherical and extends across the separation (12) or opening (30) between the arms (17,18) of the backing.

## Patentansprüche

1. Verfahren zur Herstellung einer Hüftgelenkpfanne für eine Hüft-Totalprothese, mit einer äußeren Verstärkung (10) und einer inneren Lagerkomponente (11), wobei die Verstärkung (10) einen im wesentlichen teilkugelförmigen Hauptteil und zwei unabhängige Flügel (17, 18) umfaßt, die vom Hauptteil vorstehen und durch eine Trennung (12) oder Öffnung (30) im Rand (13) der Verstärkung (10) gebildet werden, dadurch gekennzeichnet, daß die Verstärkung (10) und die innere Lagerkomponente (11) aus synthetischem Kunststoff bestehen und zu einer einzigen Komponente zusammengeformt sind.

2. Verfahren nach Anspruch 1, dadurch gekennzeichnet, daß die Verstärkung (10) steifer ist als die innere Lagerkomponente (11).

3. Verfahren nach Anspruch 1 oder Anspruch 2, dadurch gekennzeichnet, daß die innere Lagerfläche der Lagerkomponente über einen Teil (16) im wesentlichen teilkugelförmig ist, wobei dieser Teil der Trennung (12) oder Öffnung (30) zwischen den Flügeln der Verstärkung (17, 18) mit Abstand im wesentlichen gegenüber liegt, und über ihre verbleibende innere Lagerfläche (17a, 18a) zurückgesetzt ist, wobei die Zurücksetzung an der Verbindung mit dem teilkugelförmigen Teil tangential ist.

4. Verfahren nach Anspruch 3, dadurch gekennzeichnet, daß die Zurücksetzung im Vergleich zu dem kugelförmigen Teil (16) als größerer Schnittradius ausgebildet ist, der ein Rotationszentrum umkreist.

5. Verfahren nach einem der Ansprüche 1 bis 4, dadurch gekennzeichnet, daß die Lagerkomponente (11) unabhängige Flügel (20, 21) ähnlich wie die Verstärkung (10), aufweist, mit einer dazwischen liegenden Trennung (12) oder Öffnung (30).

6. Verfahren nach Anspruch 5, dadurch gekennzeichnet, daß die Lagerkomponente (11) im wesentlichen die gleiche Gestalt hat wie die Verstärkung (10).

7. Verfahren nach einem der Ansprüche 1 bis 4, dadurch gekennzeichnet, daß die Lagerkomponente (11) im wesentlichen halbkugelförmig ist und sich zwischen den Flügeln (17, 18) der Verstärkung über die Trennung (12) oder Öffnung (30) erstreckt.

## Revendications

1. Procédé de fabrication d'une cupule acétabulaire pour prothèse totale de la hanche, comportant un revêtement extérieur (10) et un constituant intérieur de support (11), ledit revêtement (10) comportant une partie principale sensiblement en partie de sphère et deux bras indépendants (17, 18) faisant saillie à partir de celle-ci et formés par une séparation (12) ou une ouverture (30) située dans le bord (13) dudit revêtement (10), caractérisé en ce que ledit revêtement (10) et ledit constituant intérieur de support (11) sont constitués de matière plastique synthétique et sont moulés ensemble pour former un constituant unique.

2. Procédé selon la revendication 1, caractérisé en ce que ledit revêtement (10) est plus rigide que le constituant intérieur de support (11).

3. Procédé selon la revendication 1 et 2, caractérisé en ce que la surface intérieure de support du constituant de support est sensiblement en partie de sphère sur une portion (16) de celle-ci espacée de manière sensiblement opposée à la séparation (12) ou à l'ouverture (30) existant entre les bras du revêtement (17, 18) et est évidée sur sa surface intérieure de support restante (17a, 18a), ledit évidement étant tangentiel à la jonction avec ladite portion en partie de sphère.

4. Procédé selon la revendication 3, caractérisé en ce que ledit évidement est formé sous forme d'un rayon de découpe plus grand que ladite portion sphérique (16) et qui a gravité autour d'un centre de rotation.

5. Procédé selon l'une quelconque des revendications 1 à 4, caractérisé en ce que le constituant de support (11) a des bras indépendants (20, 21) de manière analogue au revêtement (10), avec une séparation (12) ou une ouverture (30) entre eux.

6. Procédé selon la revendication 5, caractérisé en ce que ledit constituant de support (11) a sensiblement la même configuration que le revêtement (10).

7. Procédé selon l'une quelconque des revendications 1 à 4, caractérisé en ce que le constituant de support (11) est sensiblement hémisphérique et s'étend à travers la séparation (12) ou l'ouverture (30) existant entre les bras (17, 18) du revêtement.
